# EUROPÄISCHE PATENTSCHRIFT

(11) **EP 1 834 600 B1**
(45) Veröffentlichungstag und Bekanntmachung des Hinweises auf die Patenterteilung: **29.02.2012**
(21) Anmeldenummer: 07005106.5
(22) Anmeldetag: 13.03.2007
(51) Int. Cl.: A61B 19/00, B08B 9/00

(54) **Vorrichtung zum Reinigen einer flexiblen Hohlwelle eines medizinischen Instruments**
Device for cleaning a flexible quill shaft for a medical instrument
Dispositif destiné au nettoyage d'un arbre creux flexible d'un instrument médical

(30) Priorität: 15.03.2006 DE 102006013980
(43) Veröffentlichungstag der Anmeldung: 19.09.2007
(73) Patentinhaber: Karl Storz GmbH & Co. KG, 78532 Tuttlingen (DE)
(72) Erfinder: Efinger, Andreas, 78604 Rietheim (DE); Hermle, Rainer, 78559 Gosheim (DE)
(74) Vertreter: Weller, Wolfgang

(56) Entgegenhaltungen:
- EP-A- 1 398 004
- US-B2- 6 824 751

## Beschreibung

Die Erfindung betrifft eine Vorrichtung zum Reinigen einer flexiblen Hohlwelle eines medizinischen Instrumentes, wobei die Vorrichtung einen Hohlraum aufweisenden Körper aufweist, in dem zumindest ein Abschnitt einer zu reinigenden flexiblen Hohlwelle aufgenommen ist, wobei die Hohlwelle ein proximales Ende, ein distales Ende und eine Wandung aufweist, die mit Öffnungen versehen ist, wobei in den Hohlraum über einen Anschluss ein Reinigungsmittel zuführbar ist, wobei eine Innenwand des Hohlraumes sich in radialem Abstand zur Außenseite der Hohlwelle befindet, und wobei axial voneinander beabstandete Strömungswiderstandsmittel vorhanden sind, die einen Raum zwischen der Außenseite der Hohlwelle und der Innenwand des Hohlraumes in axialer Richtung begrenzen.

Ein chirurgisches Instrument, das eine derartige flexible bzw. biegsame Hohlwelle aufweist, ist aus der EP 0 986 989 A1 bekannt.

Aufgrund der Flexibilität bzw. der Biegsamkeit der Hohlwelle kann das Instrument gekrümmt bzw. gebogen ausgebildet sein. Die flexible Hohlwelle ist in dem gekrümmten Schaft aufgenommen und ist proximalseitig mit einem Antrieb verbunden, der die Hohlwelle in dem gekrümmten Schaft dreht. Am distalen Ende ist die Hohlwelle mit einem Werkzeug, beispielsweise mit einer Schneide oder einem Fräskopf, versehen. Die Wandung der Hohlwelle ist entweder mit einer einzigen sich wendelnden Öffnung oder mit zahlreichen Öffnungen versehen, durch die die Flexibilität erreicht wird. Diese Öffnung besteht beispielsweise aus einem mäanderförmigen Einschnitt in der Wandung, der längs einer schraubenförmigen Linie verläuft. Dadurch ist es möglich, dass bei gekrümmter und sich drehender Hohlwelle jeweils an der Außenseite der Krümmung die Öffnungen etwas aufweiten können.

Wie zuvor erwähnt, dient die Hohlwelle im medizinischen Instrument dazu, in einem Körper Gewebe abzutragen oder an einem Knochen Abfräsungen vorzunehmen. Dabei kommen kontaminierende Flüssigkeiten, wie Blut oder sonstige Gewebeflüssigkeiten, mit der Hohlwelle in Berührung. Durch die Öffnungen bzw. Schlitze in der Wandung der Hohlwelle können diese kontaminierenden Flüssigkeiten in das Innere der Hohlwelle eindringen.

Beim Reinigen der Hohlwelle ist diese üblicherweise vom Instrument abgenommen und erstreckt sich geradlinig.

Für lang erstreckte endoskopische Geräte schlägt die US 6,824,751 B2 eine Ausgestaltungsform einer Vorrichtung zum Reinigen vor, die die Form einer Röhre aufweist.

Die Erfindung der US 6,824,751 B2 bezieht sich in erster Linie auf ein Verfahren für Reinigungen in Kammern, wie z.B. einer Röhre, wobei es dabei um Reinigungsverfahren geht, die auf Verwirbelungen beruhen, die durch einen Kavitationsgenerator erzeugt werden.

In der zuvor genannten Röhre, die auf einer Seite eine Öffnung aufweist, kann das stabförmige Ende eines Endoskops von dieser Seite her eingeführt werden. Durch einen Zugangskanal am Endoskop wird die Röhre mit der Reinigungsflüssigkeit befüllt. Über eine Öffnung in der Außenwand der Röhre, wird die Reinigungsvorrichtung mit dem Kavitationsgenerator verbunden. Dieser sorgt dann mittels bestimmter Druckschwankungen dafür, dass das Endoskop außen durch die so verursachten Verwirbelungen in der Reinigungsflüssigkeit gereinigt wird.

Eine ähnliche Vorrichtung in Form einer Röhre wird in der EP 1 398 004 A2 beschrieben. Dabei wird eine Reinigungsflüssigkeit zunächst auf einzelne Düsenkanäle verteilt und strömt dann durch diese Düsenkanäle auf die Außenseite des zu reinigenden Instruments.

Um einen kontinuierlichen Strom an Reinigungsflüssigkeit zu erhalten, besitzt diese Röhre zwei Öffnungen, jeweils eine an einem Ende. Durch die eine Öffnung wird das Instrument eingeschoben und durch die andere Öffnung kann die Reinigungsflüssigkeit ungehindert abfließen.

Zum Reinigen der Hohlwelle müssen nicht nur deren Innen- und Außenseite gereinigt werden, sondern auch die Öffnungen, insbesondere die mäanderförmigen Schlitze, deren Breite im Bereich von etwa 0,05 bis 1 mm liegt.

Dies hat sich in der Praxis als äußerst umständlich und schwierig erwiesen.

Es ist daher Aufgabe der vorliegenden Erfindung, eine Möglichkeit zu schaffen, um eine flexible Hohlwelle einfach und effektiv zu reinigen.

Erfindungsgemäß wird die Aufgabe dadurch gelöst, dass die Hohlwelle sowohl mit ihren proximalen als auch distalen Ende aus dem Raum in axialer Richtung hinausragt, und dass die axial voneinander beabstandeten Strömungswiderstandsmittel so ausgestaltet sind, dass der Strömungswiderstand der Öffnungen der flexiblen Hohlwelle bei entsprechender Druckbeaufschlagung des Reinigungsmittels, durch das Reinigungsmittel überwindbar ist, so dass das zugeführte Reinigungsmittel über die Öffnungen in der Wandung der Hohlwelle in deren Innenraum eindringen kann, wodurch zumindest ein Teil des Reinigungsmittels den Innenraum der Hohlwelle reinigt und durch die Hohlwelle abgeführt werden kann.

In eine Vorrichtung mit diesen konstruktiven Merkmalen kann eine zu reinigende Hohlwelle eingeschoben werden. Da der radiale Durchmesser des Hohlraumes im Inneren des Körpers größer ist als der Außendurchmesser der Hohlwelle, existiert um die Außenseite der Hohlwelle herum ein Raum, der von der Außenseite her über den Anschluss mit einem Reinigungsmittel beaufschlagt werden kann. Dadurch, dass axial voneinander beabstandete Strömungswiderstandsmittel vorhanden sind, die diesen Raum zur Außenseite hin axial begrenzen, kann das zugeführte Reinigungsmittel über die Öffnungen in der Wandung der Hohlwelle in deren Innenraum eindringen. Dadurch wird nicht nur die Außenseite der Hohlwelle gereinigt, sondern auch die Öffnungen bzw. Schlitze, indem das Reinigungsmittel von außen nach innen in die Hohlwelle eindringt. Durch entsprechende Druckbeaufschlagung des Reinigungsmittels kann der Strömungswiderstand der meist mäanderförmigen Schlitze entsprechend überwunden werden. Das in den Innenraum der Hohlwelle eintretende Reinigungsmittel wird anschließend aus der Hohlwelle abgeführt, sei es an den endseitig über die Vorrichtung überstehenden Enden der Hohlwelle oder über spezielle Öffnungen in der Wand der Hohlwelle, die axial außerhalb der Strömungswiderstandsmittel liegen. Dabei werden nicht nur die Verunreinigungen abgeführt, sondern es wird gleichzeitig noch die Innenseite bzw. die Innenwand der Hohlwelle gereinigt.

Das Reinigungsmittel kann eine Reinigungsflüssigkeit sein. Es kann auch in Form von Gasen, z.B. Reinigungsluft, zugeführt werden. Es können auch Mischungen aus einem Trägermittel und einem Reinigungsmittel, unabhängig von deren jeweiligem Aggregatszustand, zugeführt werden.

Durch die erfindungsgemäße Vorrichtung kann nun ein Abschnitt der Hohlwelle vollständig gereinigt werden, d.h. deren Außenseite, die Durchbruchstellen in der Wandung und deren Innenseite. Dadurch ist eine besonders einfache und effektive Reinigung einer solchen Hohlwelle insbesondere im Bereich von deren Durchbruchstellen zu erzielen, da an diesen Stellen das Reinigungsmittel radial von außen nach innen strömt und kontaminierende Flüssigkeiten oder sonstige Verunreinigungen abspült.

In einer weiteren Ausgestaltung der Erfindung ist der Körper der Vorrichtung als Hohlzylinder ausgebildet.

Diese Maßnahme hat den Vorteil, dass die Vorrichtung eine schlank bauende Konstruktion darstellt, in deren Innenraum die Hohlwelle zur Reinigung eingeschoben werden kann, was sehr einfach durchzuführen ist und den Reinigungsvorgang zusätzlich erleichtert.

In einer weiteren Ausgestaltung der Erfindung verläuft der Körper geradlinig.

In dieser Ausgestaltung ist die Hohlwelle entsprechend langerstreckt, und die Öffnungen in deren Wandung, meist die über einen Laserstrahl eingeschnittenen mäanderförmigen, schraubenlinienförmig verlaufende Schlitze, haben durchgehend die gleiche Breite, so dass dem einströmenden Reinigungsmittel ein gleichmäßiger Strömungswiderstand entgegengestellt wird, was dazu führt, dass das Reinigungsmittel gleichmäßig verteilt durch die Öffnungen in den Innenraum der Hohlwelle eintritt.

In einer weiteren Ausgestaltung der Erfindung verläuft der Körper gekrümmt.

Ein gekrümmter Körper der Vorrichtung hat den Vorteil, dass im äußeren Umfang der Krümmung die Öffnungen bzw. Schlitze der Hohlwelle etwas gespreizt werden, so dass dem eintretenden Reinigungsmittel in diesem Bereich ein etwas geringerer Widerstand entgegengebracht wird. Allerdings sollte dann die Hohlwelle in der Vorrichtung gedreht werden, damit alle Schlitze nach und nach gleichmäßig gereinigt werden.

Diese Ausgestaltung kann dann wünschenswert sein, wenn zu befürchten ist, dass kleinste feine Partikel sich in den Schlitzen festsetzen, die durch den Druck des Reinigungsmittels nicht aus den Schlitzen herausgespült werden können, wenn beispielsweise diese feinen Partikel eine keilförmige Kontur aufweisen. Dann ist es hilfreich, wenn in dem gekrümmt verlaufenden Körper zumindest zeitweilig die Schlitze etwas gespreizt werden, so dass festsitzende Feststoffpartikel ebenfalls in den Innenraum abgespült werden können. Dies ist insbesondere hilfreich, wenn das Werkzeug der Hohlwelle als Fräskopf ausgebildet ist.

In einer weiteren Ausgestaltung der Erfindung entspricht der axiale Abstand der Strömungswiderstandsmittel in etwa der Länge des Abstandes der Hohlwelle, der mit den Öffnungen versehen ist.

Diese Maßnahme hat den Vorteil, dass in einem einzigen Arbeitsvorgang nahezu sämtliche Öffnungen über den Längsabschnitt der Hohlwelle gereinigt werden können.

In einer Ausgestaltung der Erfindung sind die Strömungswiderstandsmittel als Dichtungsmittel ausgebildet.

Diese Maßnahme hat den Vorteil, dass das in den Zwischenraum zwischen Innenseite des Hohlraumes und Außenseite der Hohlwelle über den Anschluss zugeführte Reinigungsmittel in seiner Gesamtheit durch die Wandung der Hohlwelle durchtreten muss. Durch die Ausgestaltung der Strömungswiderstandsmittel als Dichtungsmittel ist ja ein axiales Ausströmen des Reinigungsmittels zwischen Außenseite der Hohlwelle und Innenseite des Hohlraumes in axialer Richtung gehindert. Da die Dichtungsmittel mit der Außenseite der zu reinigenden Hohlwelle an den Dichtstellen dichtend in Berührung stehen, ist ein Reinigen an diesen Stellen nur dann möglich, wenn die Lage der Hohlwelle während des Reinigungsvorgangs zumindest einmal verändert wird, so dass Stellen, die anfänglich durch die Dichtungsmittel belegt sind, ebenfalls gereinigt werden können.

In einer weiteren Ausgestaltung der Erfindung ist der Anschluss zum Zuführen des Reinigungsmittels etwa mittig im Körper der Vorrichtung angeordnet.

Diese Maßnahme hat den Vorteil, dass eine beidseits des Anschlusses gleichmäßige Durchströmung des mit Öffnungen versehenen Körpers der Hohlwelle bewerkstelligt werden kann und Druckverluste in axialer Richtung vernachlässigbar sind.

In einer weiteren Ausgestaltung der Erfindung ist die Hohlwelle relativ zum Körper verschiebbar.

Diese relative Verschiebbarkeit kann nun so erfolgen, dass entweder der Körper stationär ist und die Hohlwelle im Körper verschoben wird, oder umgekehrt, dass die Hohlwelle stationär ist und der Körper über diese hinweg verschoben wird, oder dass beide verschoben werden.

Dies ist insbesondere dann erwünscht, wenn relativ lange Hohlwellen gereinigt werden sollen und die Vorrichtung zum Reinigen, sei es aus Staugründen oder aus sonstigen Gründen, nur ein relativ kurzes axiales Maß einnehmen soll.

In diesem Fall wird beispielsweise die Hohlwelle in den Körper ein- und durch diesen hindurchgeschoben, so dass letztendlich jeweils nur ein gewisser Teilabschnitt der Hohlwelle in der Vorrichtung aufgenommen ist. Da im Falle von Dichtungen diese den Abschnitt zur Außenseite hin abdichten, kann das Reinigungsmittel nur in diesem begrenzten Abschnitt in den Innenraum der Hohlwelle eindringen. Durch die Relativverschiebung kann nun nach und nach der gesamte axiale Längenabschnitt mit dem Reinigungsmittel beaufschlagt und gereinigt werden, wobei dies durch die zuvor erwähnte Relativverschiebung erfolgt.

In einer weiteren Ausgestaltung der Erfindung ist der axiale Abstand der Dichtungsmittel untereinander veränderbar.

Diese Maßnahme hat den Vorteil, dass je nach Ausgestaltung der Hohlwelle und der zur Verfügung stehenden Druckbeaufschlagung des Reinigungsmittels unterschiedliche axiale Abschnitte gereinigt werden können.

In einer weiteren Ausgestaltung der Erfindung sind die Dichtungsmittel als O-Ringe ausgebildet.

Diese Maßnahme hat den Vorteil, dass kostengünstige und effektive Dichtungsmittel vorgesehen sind, um den Raum zwischen der Außenseite der Hohlwelle, die ja meist zylindrisch ist, und dem Innenraum des Körpers der Vorrichtung abzudichten. Die Konstruktion als O-Ring erlaubt auch sehr einfach die relative Verschiebbarkeit zwischen Vorrichtung und Hohlwelle bzw. die Verschiebbarkeit der Dichtungsmittel untereinander in axialer Richtung.

In einer weiteren Ausgestaltung der Erfindung sind die Dichtungsmittel in axial verschiebbaren Lagerbuchsen aufgenommen.

Diese Maßnahme hat den Vorteil, dass über diese Lagerbuchsen die Dichtungsmittel in ihrem axialen Abstand untereinander veränderbar sind.

Dazu sind die Lagerbuchsen, die die Dichtungen, beispielsweise die O-Ringe, halten, axial verschiebbar im Inneren des Körpers aufgenommen.

Diese Verschiebbarkeit kann nun von außen mechanisch von Hand erfolgen, beispielsweise wenn unterschiedlich lange Abschnitte einer Hohlwelle gereinigt werden sollen, oder die Verschiebbarkeit kann durch Regulierung des Druckes der Spülflüssigkeit erreicht werden.

Dies eröffnet die Möglichkeit, zu Beginn eines Spülvorganges die Dichtungsmittel in einem relativ kurzen axialen Abstand voneinander anzuordnen und in diesem Zustand zunächst das Reinigungsmittel einzuführen. Der Druck kann nun so gewählt werden, dass ein Teil des auf das Reinigungsmittel ausgeübten Drucks dazu dient, die Dichtungsmittel in axialer Richtung gegensinnig zueinander zu verschieben, so dass nach und nach ein immer größerer Abschnitt des Körpers der Hohlwelle durchspült wird.

Dies zeigt die flexible Ausgestaltung der erfindungsgemäßen Vorrichtung, um an unterschiedliche Konstruktionen und Formen von Hohlwellen anpassen zu können.

In einer weiteren Ausgestaltung der Erfindung sind die Strömungswiderstandsmittel dadurch gebildet, dass der radiale Abstand der Innenwand des Hohlraumes zur Außenseite der Hohlwelle im Bereich der Strömungswiderstandsmittel geringer ist als im Bereich zwischen diesen axial voneinander beabstandeten Stellen.

Diese Maßnahme hat den Vorteil, dass nunmehr nicht, wie zuvor beschrieben, eine völlige Abdichtung an den axial voneinander beabstandeten Stellen stattfindet, sondern dass die Strömungswiderstandsmittel durch einen radial verengten Raum gebildet sind.

Dies eröffnet die Möglichkeit, dass ein Teil des Reinigungsmittels auch in diesem Bereich des radial verengten Raumes zwischen der Innenseite des Hohlraumes und der Außenseite der Hohlwelle in axialer Richtung durch- und austreten kann. Somit ist es auch möglich, in diesem Bereich der Strömungswiderstandsmittel Reinigungsmittel hindurchzutransportieren und die Außenseite zu reinigen. Dadurch ist es auch möglich, die Vorrichtung so zu betreiben, dass keine Relativverschiebung zwischen Hohlwelle und Vorrichtung notwendig ist, da das Reinigungsmittel die gesamte Außenseite der Hohlwelle überströmen kann. Sollte in diesem Bereich eine Öffnung der Wandung liegen, kann das Reinigungsmittel in radialer Richtung durch die Wandung durchtreten und die Öffnung reinigen.

In einer weiteren Ausgestaltung der Erfindung wird dies konstruktiv dadurch erreicht, dass im Bereich eines Strömungswiderstandsmittels ein Ringspalt zwischen der Innenwand des Hohlraumes und der Außenseite der Hohlwelle zum axialen Durchtritt von Reinigungsmittel vorhanden ist.

Diese Maßnahme hat den Vorteil, dass ein solcher Ringspalt eine genau definierte geometrische Größe darstellt, die dem Reinigungsmittel einen genau bestimmten, berechenbaren Strömungswiderstand entgegenstellt. Durch diese Geometrie ist auch eine Selbstzentrierung der Hohlwelle in dem Ringspalt möglich, da das durch den Ringspalt hindurchtretende, unter Druck stehende Reinigungsmittel gleichmäßig verteilt durch den Ringspalt strömen wird, wodurch die Zentrierung erreicht wird. Somit ist sichergestellt, dass alle Bereiche der Außenseite der Hohlwelle, die im Bereich eines Ringspaltes zum Liegen kommen, von hindurchtretendem Reinigungsmittel beaufschlagt und gespült werden.

In einer weiteren Ausgestaltung der Erfindung ist Reinigungsmittel zusätzlich dem radial verengten Raum, z.B. den Ringspälten, unmittelbar zuführbar.

Aufgrund der üblichen Größen von solchen Hohlwellen liegen die Ringspaltbreiten im Bereich von Bruchteilen von Millimetern.

Wird eine an ihrer Außenseite kontaminierte Hohlwelle in eine Vorrichtung eingeschoben, kann nicht ausgeschlossen werden, dass Verunreinigungen, wie Blut, Gewebe oder Knochensplitter, in diesen Spalt, also den Raum zwischen Außenseite der Hohlwelle und verengter Innenseite des Hohlraumes, eingeschoben werden und sich dort festsetzen. Es ist auch möglich, dass im Betrieb die zwischen den axial beabstandeten Stellen im durchmessergrößeren Bereich des Hohlraumes abgespülten Teile in axialer Richtung längs der Außenseite bewegt werden, in die Ringspälte eintreten und diese an gewissen umfänglichen Stellen oder auch vollständig verstopfen.

Durch das Vorsehen der Möglichkeit, Reinigungsmittel unmittelbar diesen Ringspälten zuzuführen, können diese von solchen Verunreinigungen freigespült werden.

In einer weiteren Ausgestaltung wird dies konstruktiv sehr einfach dadurch bewerkstelligt, dass vom Anschluss zumindest ein Bypass abzweigt, der im Bereich eines Ringspaltes mündet.

Dadurch ist es möglich, ständig einen Teil des der Vorrichtung zugeführten Reinigungsmittels über den Bypass abzuzweigen und direkt dem Ringspalt druckbeaufschlagt durch den Bypass und zusätzlich von außen zuzuführen, wodurch ein Abspülen von Verunreinigungen zusätzlich ermöglicht wird.

In einer weiteren Ausgestaltung der Erfindung ist eine zu reinigende Hohlwelle nur von einer ersten Seite her in den Hohlraum einführbar.

Diese Maßnahme zeigt sich bei den Konstruktionen mit den Ringspälten dahingehend vorteilhaft, dass insbesondere der Ringspalt an der Einschubseite den zuvor beschriebenen Verunreinigungen ausgesetzt ist, so dass es dann ausreichend ist, nur an einer Seite, nämlich dieser Einschubseite, die zusätzlichen konstruktiven Aufwendungen eines Bypasses vorzusehen.

In einer weiteren Ausgestaltung der Erfindung ist an der ersten Seite ein Anschlag vorhanden, an den eine entsprechende Anlage einer Hohlwelle anlegbar ist.

Diese Maßnahme hat den Vorteil, dass eine genau definierte Position der Hohlwelle relativ zum Körper der Vorrichtung vorgegeben ist, so dass die zuvor erwähnten Spülvorgänge über den Ringspalt einwandfrei stattfinden können. Dies eröffnet auch die Möglichkeit, die Hohlwelle so weit in die Vorrichtung einzuschieben, dass sämtliche in der Wandung der Hohlwelle vorhandenen Schlitze im Inneren des Hohlraumes liegen, so dass mit einem einzigen Spülvorgang bei einer einzigen Relativstellung zwischen Vorrichtung und Hohlwelle diese vollständig gereinigt werden kann. Dies ermöglicht eine vollautomatische Durchführung, so dass anfänglich lediglich die Hohlwelle bis zu dem Anschlag in die Vorrichtung eingeschoben werden muss und anschließend dieser Zusammenbau in einer automatisierten Waschanlage eingesetzt und mit Reinigungsmittel beaufschlagt werden kann.

In einer weiteren Ausgestaltung der Erfindung kommt an der ersten Seite ein distales Ende der Hohlwelle zum Liegen.

Diese Maßnahme hat den Vorteil, dass die Hohlwelle mit dem proximalen Ende, das meist weniger verunreinigt ist, zunächst von der ersten Seite her eingeschoben wird. Nach vollständigem Einschieben der Hohlwelle kommt der wesentlich stärker kontaminierte distale Bereich der Hohlwelle an der ersten Seite zum Liegen und kann dort besonders gründlich gereinigt werden.

In einer weiteren Ausgestaltung der Erfindung weist der Körper an einer zweiten, der ersten Seite gegenüberliegenden Seite eine Öffnung auf, in der das proximale Ende der Hohlwelle zum Liegen kommt.

Diese Maßnahme hat den Vorteil, dass im Bereich des zweiten Endes ebenfalls Reinigungsmittel austreten kann. Je nach Konstruktion, also mit Ringspälten oder mit Dichtungsmitteln, tritt über diese Öffnung Reinigungsmittel aus, das nur in das Innere der Hohlwelle eingedrungen ist, oder es tritt auch noch zusätzlich Reinigungsmittel aus, das über den Ringspalt über die Außenseite der Hohlwelle entlanggeführt wird.

In einer weiteren Ausgestaltung der Erfindung weist der Körper zumindest im Bereich der ersten Seite an dessen Außenseite flüssigkeitsleitende Merkmale auf, die auf die Außenseite auftreffende Reinigungsflüssigkeit gezielt auf die erste Seite zuführen.

Diese Maßnahme hat den Vorteil, dass die erste Seite, also die Einführseite, gezielt zusätzlich mit Reinigungsflüssigkeit beaufschlagt wird, die auf die Außenseite der Vorrichtung trifft, beispielsweise wenn diese in einer mit Sprüharmen versehenen automatischen Waschmaschine aufgenommen ist.

Die Hohlwelle muss von einer Seite der Vorrichtung her eingeschoben werden. Unabhängig davon, ob die Strömungswiderstandsmittel als Dichtungsmittel oder als Ringspälte ausgebildet sind, werden größere an der Außenseite der Hohlwelle anhaftende Verunreinigungen beim Einführen längs der Außenseite der Hohlwelle zu dem nacheilenden Ende hin verschoben. Dadurch kann es zu einer Anhäufung von solchen Verunreinigungen im Bereich der Einführseite der Vorrichtung kommen. Durch die gezielte Zuführung der Reinigungsflüssigkeit von der Außenseite zu dieser Stelle hin können solche äußeren anhaftenden Verunreinigungen vollständig abgespült werden.

In einer weiteren Ausgestaltung der Erfindung ist im Hohlraum im Abstand zu dessen Innenwand eine Zwischenwand angeordnet, die im Abstand zur Außenseite der Hohlwelle liegt, und in der Zwischenwand sind Durchtrittsöffnungen vorhanden.

Diese Maßnahme hat den Vorteil, dass durch die Öffnungen in der Zwischenwand das Reinigungsmittel ganz gezielt der Außenseite der Hohlwelle zugeführt werden kann. Bei den Ausgestaltungen, bei denen die Strömungswiderstandsmittel als schmale Ringspälte ausgebildet sind, aus denen Reinigungsmittel austreten kann, kann durch Vorsehen von Öffnungen in diesem Bereich ganz gezielt Reinigungsmittel zugeführt werden. Das Reinigungsmittel strömt von außen zunächst in den äußeren Hohlraum zwischen der Innenseite des Hohlraums und der Innenwand und kann sich dort gleichmäßig verteilen. Über die Öffnungen in der Innenwand wird das Reinigungsmittel dann, wie zuvor erwähnt, gezielt in den inneren Hohlraum, in dem die Hohlwelle angeordnet ist, zugeführt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine Seitenansicht einer Hohlwelle, die mit der erfindungsgemäßen Vorrichtung gereinigt werden soll,
- Fig. 2: einen Längsschnitt der Hohlwelle von Fig. 1,
- Fig. 3: einen Längsschnitt eines ersten Ausführungsbeispiels einer Vorrichtung zum Reinigen der Hohlwelle von Fig. 1 und 2,
- Fig. 4: ein den Fig. 2 und 3 vergleichbaren Schnitt, wobei die Hohlwelle von Fig. 2 in die Vorrichtung zum Reinigen von Fig. 3 eingeschoben ist,
- Fig. 5: einen stark vergrößerten Ausschnitt des linken Endes des Schnittes von Fig. 4,
- Fig. 6: eine der Darstellung von Fig. 5 vergleichbare Darstellung eines weiteren Ausführungsbeispiels einer Vorrichtung mit Ringspälten, in der eine Hohlwelle von Fig. 1 eingeschoben ist,
- Fig. 7: eine der Darstellung von Fig. 6 vergleichbare Darstellung eines weiteren Ausführungsbeispiels mit einem Bypass für die Reinigungsflüssigkeit,
- Fig. 8: eine Draufsicht auf einen gegenüberliegenden Endbereich der in Fig. 6 und/oder Fig. 7 gezeigten Varianten,
- Fig. 9: einen Schnitt längs der Linie IX-IX in Fig. 8,
- Fig. 10: einen Längsschnitt eines weiteren Ausführungsbeispiels einer erfindungsgemäßen Vorrichtung, in der eine Hohlwelle eingeschoben ist,
- Fig. 11: eine stark vergrößerte Schnittdarstellung des linken Endbereiches des Schnittes von Fig. 10, und
- Fig. 12: einen Schnitt längs der Linie XII-XII in Fig. 11.

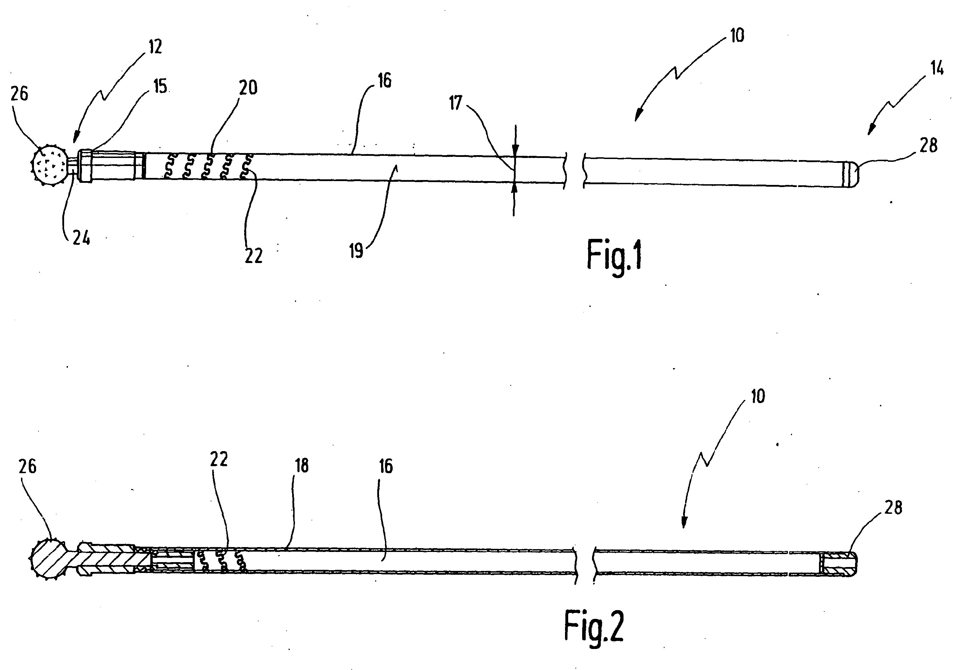

Eine in den Fig. 1 und 2 dargestellte Hohlwelle ist in ihrer Gesamtheit mit der Bezugsziffer 10 versehen.

Die Hohlwelle 10 weist ein distales Ende 12 und ein proximales Ende 14 auf. Die Hohlwelle 10 ist als Rohr 16 ausgebildet, das sich zwischen distalem Ende 12 und proximalem Ende 14 erstreckt und mit diesen fest verbunden ist.

In der Wandung 18 des Rohres 16 sind zahlreiche Öffnungen 20 vorgesehen.

Im dargestellten Ausführungsbeispiel sind die Öffnungen 20 als ein mäanderförmiger Schlitz 22 ausgebildet, der sich etwa vom distalen Ende 12 bis zum proximalen Ende 14 schraubenlinienförmig windet (in den Figuren ist nur ein Teilabschnitt dargestellt). Durch diesen Schlitz 22 ist das Rohr 16 um Achsen senkrecht zu seiner Längsachse biegsam, kann aber gleichzeitig Drehkräfte um seine Längsachse übertragen. Am distalen Ende 12 ist ein Werkzeug 24 angeordnet, das als Fräskopf 26 ausgebildet ist. Am proximalen Ende 14 ist eine Kupplung 28 vorgesehen, die in einen Drehantrieb eines hier nicht dargestellten chirurgischen Instrumentes eingesteckt werden kann.

Die Konstruktion und die Betriebsweise eines solchen chirurgischen Instruments mit einer biegsamen Hohlwelle, die in einem gekrümmten Schaft des chirurgischen Instrumentes drehbar aufgenommen ist, ist insbesondere in der EP 0 986,989 A1 beschrieben, auf die hier ausdrücklich Bezug genommen wird. Dabei steckt die Hohlwelle 10 etwa bis zu dem Anschlag 15 (siehe Fig. 1) im Inneren eines gekrümmten Schaftes des chirurgischen Instrumentes, lediglich der Fräskopf 56 schaut distalseitig hervor.

Beim Einsatz können nun Verunreinigungen, wie Blut, Gewebeflüssigkeit oder feiner Knochenabrieb, über die Schlitze 22 in das Innere der Hohlwelle 10 eintreten, so dass nach einem chirurgischen Eingriff eine entsprechende Reinigung notwendig ist.

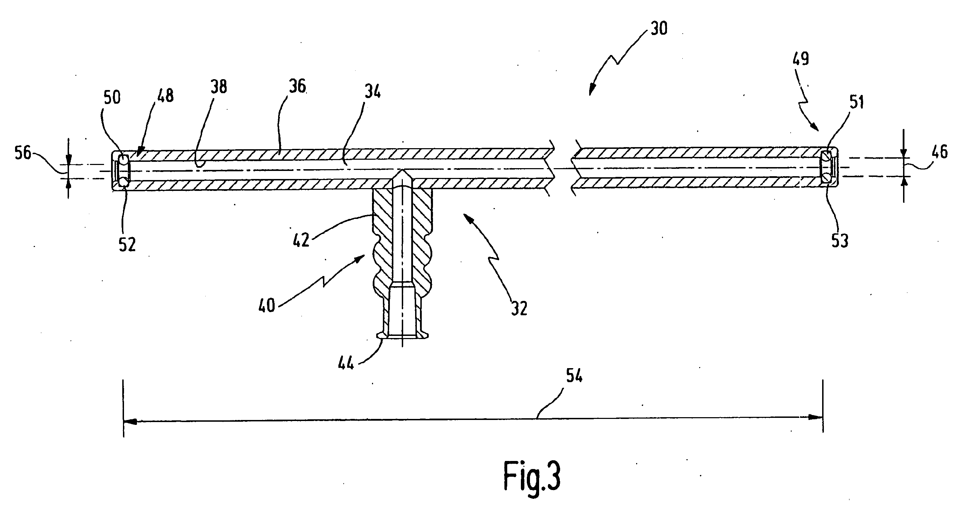

Dazu ist die erfindungsgemäße Vorrichtung zum Reinigen vorgesehen, die in Fig. 3 in ihrer Gesamtheit mit der Bezugsziffer 30 versehen ist.

Die Vorrichtung 30 weist einen Körper 32 auf, der einen inneren Hohlraum 34 aufweist. Der Körper 32 weist einen Hohlzylinder 36 auf, der eine entsprechende zylindrische Innenwand 38 aufweist.

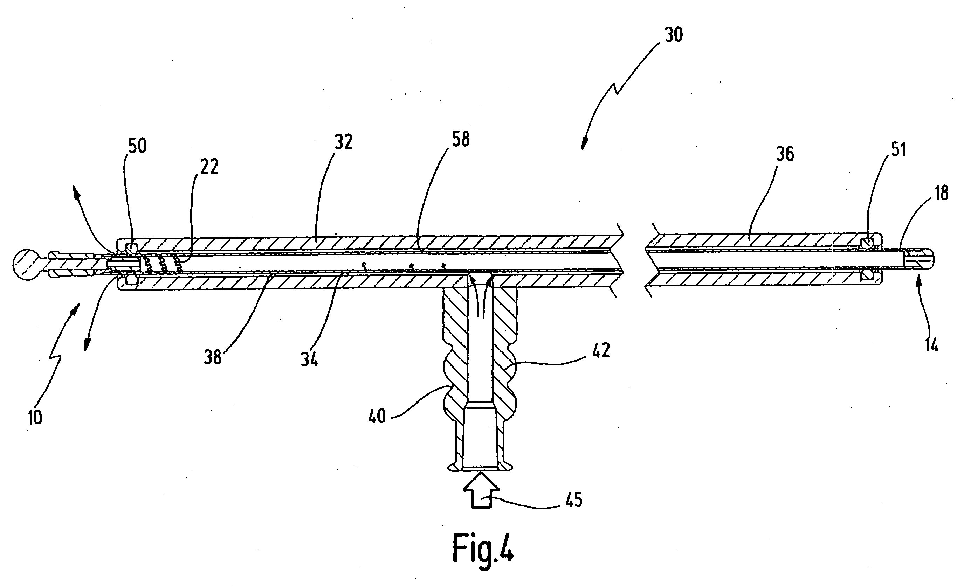

Über einen mittigen, etwa rechtwinklig seitlich abstehenden Anschluss 40 kann der Hohlraum 34 z.B. mit einer Reinigungsflüssigkeit beaufschlagt werden, wie das nachfolgend noch im Zusammenhang mit Fig. 4 beschrieben wird.

Der Anschluss 40 ist als seitlich abstehender Stutzen 42 ausgebildet, der an seinem äußeren Ende mit einem so genannten Luer-Lock 44 versehen ist. An diesen Luer-Lock 44 kann beispielsweise eine entsprechende Spritze mit einer Reinigungsflüssigkeit oder ein Gerät mit einer Fördereinrichtung zum Fördern einer Reinigungsflüssigkeit angeschlossen werden. An den gegenüberliegenden Endbereichen des Hohlzylinders 36 sind Dichtungsmittel 48, 49 in Form von O-Ringen 50 bzw. 51 vorhanden, die in innenliegende Nuten 52 bzw. 53 eingesetzt sind. Der Innendurchmesser 46 des Hohlzylinders 36 ist so bemessen, dass er größer ist als der Außendurchmesser 17 der in Fig. 1 dargestellten Hohlwelle 10.

Der lichte Innendurchmesser 56 der O-Ringe 50, 51 ist so bemessen, dass dieser geringfügig kleiner ist als der Außendurchmesser 17 der Hohlwelle 10, dennoch aber so bemessen ist, dass die Hohlwelle 10 durch die O-Ringe 50, 51 hindurchgeschoben werden kann.

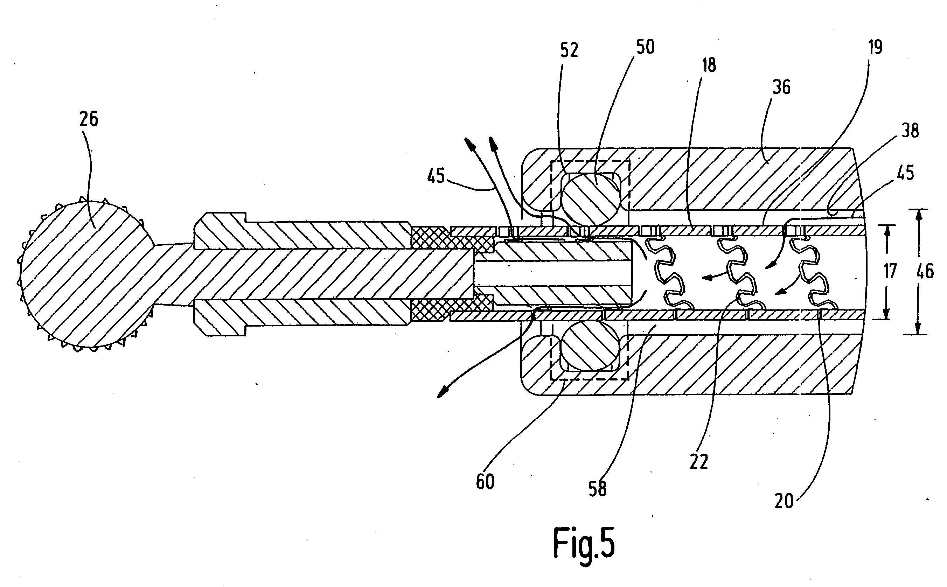

Die O-Ringe 50, 51 sind so ausgestaltet, dass bei eingeschobener Hohlwelle 10, wie dies nachfolgend im Zusammenhang mit den Fig. 4 und 5 beschrieben wird, eine Abdichtung des Ringraumes 58, der sich zwischen der Außenseite 19 der Hohlwelle 10 und der Innenwand 38 des Hohlzylinders 36 erstreckt, in axialer Richtung bewerkstelligt wird, wie dies aus Fig. 4 und 5 ersichtlich ist.

In Fig. 4 ist gezeigt, wie die Hohlwelle 10 von der linken Seite her in den Hohlzylinder 36 eingeschoben wurde, bis das proximale Ende 14 über die rechte Seite des Hohlzylinders 36 in der Darstellung von Fig. 4 herausragt.

Der axiale Abstand 54 der beiden O-Ringe 50, 51 untereinander (siehe Fig. 3) ist so bemessen, dass dieser in etwa der gesamten Längserstreckung des Rohres 16 der Hohlwelle 10 entspricht, die mit dem mäanderförmigen Schlitz 22 versehen ist; allerdings ist dieser Abstand 54 etwas geringer. Wird nun, wie in Fig. 4 dargestellt, über den Stutzen 42 eine Reinigungsflüssigkeit 45 zugeführt, verteilt sich diese, wie durch Strömungspfeile angedeutet ist, gleichmäßig in dem Ringraum 58 zwischen der Innenwand 38 des Hohlzylinders 36 und der Außenseite 19 der Hohlwelle 10. In axialer Richtung ist dieser Ringraum 58 durch die O-Ringe 50, 51 abgedichtet.

Wie insbesondere aus der vergrößerten Darstellung von Fig. 5 ersichtlich ist, strömt die unter Druck stehende Reinigungsflüssigkeit 45 entlang der Außenseite der Hohlwelle 10, so dass dort anhaftende Verunreinigungen abgespült werden können. Die Reinigungsflüssigkeit 45 kann nun über die zahlreichen Öffnungen 20, also den mäanderförmigen Schlitz 22, in den Innenraum der Hohlwelle 10 eindringen, wie dies durch die Strömungspfeile angedeutet ist. Dadurch werden diese Öffnungen 20 gereinigt. Die Reinigungsflüssigkeit 45 strömt dann im Inneren der Hohlwelle 10 zu den gegenüberliegenden Enden und tritt dort über Öffnungen 20 in der Hohlwelle 10 bzw. die Bereiche des Schlitzes 22 außerhalb der O-Ringe aus.

Wie aus der vergrößerten Darstellung von Fig. 5 ersichtlich, bestehen diese Austrittsöffnungen zum Austreten der Reinigungsflüssigkeit aus dem Endabschnitt des mäanderförmigen Schlitzes 22, der sich axial außerhalb der Dichtstelle durch den O-Ring 50 befindet.

Es kann allerdings auch vorgesehen sein, dass ganz speziell ausgerichtete Auslassbohrungen in der Hohlwelle 10 vorgesehen sind, um die Reinigungsflüssigkeit gezielt an bestimmten Stellen austreten zu lassen.

Aus Fig. 4 ist ersichtlich, dass hier eine Art "stationäre" Reinigung durchgeführt wird, d.h. die Länge des Hohlzylinders 36 entspricht in etwa der Länge des geschlitzten Rohres 16 der Hohlwelle 10.

Ist nun die Hohlwelle 10 wesentlich länger, oder ist die Länge des Hohlzylinders 36 wesentlich kürzer, kann dennoch eine Reinigung bewerkstelligt werden, wozu dann eine Relativverschiebung zwischen Hohlwelle 10 und Vorrichtung 30 notwendig ist.

Diese Relativverschiebung kann nun dadurch erfolgen, dass entweder die Hohlwelle 10 relativ zum Hohlzylinder 36 bewegt wird, oder dass der Körper 32 über die Hohlwelle 10 hinwegbewegt wird. Man kann auch beide Bewegungen synchron durchführen.

In Fig. 5 ist eine weitere Variante angedeutet, nämlich, dass der O-Ring 50, wie auch gegebenenfalls der gegenüberliegende O-Ring 51, in einer verschiebbaren Lagerbuchse 60 aufgenommen ist. Über diese Ausgestaltung kann das jeweilige Dichtungsmittel, also beispielsweise der O-Ring 50, in axialer Richtung längs des Hohlzylinders 36 verschoben werden, wozu an der Innenseite des Hohlzylinders 36 entsprechende Ausnehmungen bzw. eine Verstellbahn für den O-Ring bewerkstelligt werden.

Diese Verschiebung der Lagerbuchse 60 kann durch die Handhabungsperson von der Außenseite durchgeführt werden, beispielsweise um die beiden O-Ringe 50, 51 samt der entsprechenden Lagerbuchse 60 sehr nahe an den Stutzen 42 heranzuführen. Durch den Druck der zugeführten Reinigungsflüssigkeit 45 können dann die Lagerbuchsen 60 voneinander weg bewegt werden, bis in eine axiale Endstellung, die beispielsweise der Darstellung von Fig. 5 entspricht.

Die Lagerbuchsen 60 können nicht nur verschiebbar sondern auch feststellbar sein, so dass der Ringraum 58 in ein beliebiges axiales Maß verstellt werden kann. Dies kann dann erwünscht sein, wenn beispielsweise nur ein bestimmter Reinigungsflüssigkeitsdruck bereitgestellt werden kann, der nur ausreichend ist, eine ausreichende Spülung über einen gewissen Längenabschnitt der Hohlwelle 10 durchzuführen. Dies ist abhängig davon, wie groß der Durchmesser ist und wie breit die Schlitze, also die Öffnungen, sind, und wie deren Geometrie ist, also wie der Strömungswiderstand ausgestaltet ist.

Dies zeigt die Flexibilität der erfindungsgemäßen Vorrichtung.

In den vorgenannten Beispielen war der Hohlzylinder 36 jeweils geradlinig ausgebildet.

Es ist auch möglich, den Hohlzylinder 36 gekrümmt auszugestalten, denn die flexible Hohlwelle 10 kann auch in einen solchen gekrümmten Hohlzylinder 36 eingeschoben werden. In diesem Falle wären am äußeren Krümmungsumfang die jeweiligen Schlitze stärker gespreizt, so dass diese der eindringenden Reinigungsflüssigkeit 45 einen geringeren Strömungswiderstand entgegenstellen würden. Allerdings muss dann dafür gesorgt werden, dass die Hohlwelle 10 in der Vorrichtung gedreht wird, um eine gleichmäßige Durchspülung der Schlitze zu gewährleisten.

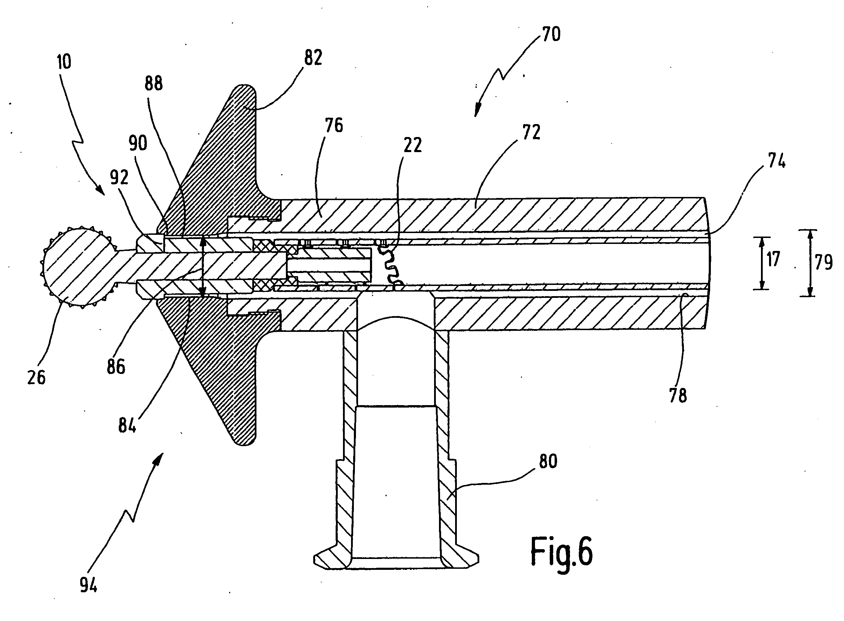

In Fig. 6 ist ein weiteres Ausführungsbeispiel einer erfindungsgemäßen Vorrichtung zum Reinigen einer Hohlwelle dargestellt, wobei die Vorrichtung in ihrer Gesamtheit mit der Bezugsziffer 70 versehen ist.

Der in Fig. 6 dargestellte vergrößerte Schnitt entspricht einem linken Endbereich der Vorrichtung, wie er in Fig. 5 dargestellt ist. Auch die Vorrichtung 70 weist einen Körper 72 auf, in dessen Innerem ein Hohlraum 74 vorhanden ist. Der Körper 72 ist, wie zuvor beschrieben, als Hohlzylinder 76 ausgebildet. Auf das in der Darstellung von Fig. 6 linke Ende ist eine Kappe 82 aufgeschraubt, die eine mittige durchgehende Öffnung 84 aufweist. Diese Öffnung 84 stellt eine Fortsetzung des Hohlraumes 74 im Körper 72 der Vorrichtung 70 dar.

Der Körper 72 ist mit einem seitlich abstehenden Anschluss 80 zum Zuführen einer Reinigungsflüssigkeit versehen, wobei in diesem Ausführungsbeispiel der Anschluss 80 nicht etwa mittig über die Länge des Körpers 72 angeordnet ist, sondern unmittelbar im Bereich dessen in Fig. 6 dargestellten Endes.

Der Durchmesser 79 der Innenwand 78 ist, wie zuvor beschrieben, größer als der Außendurchmesser 17 der in den Hohlraum 74 eingeschobenen Hohlwelle 10. Somit kann, wie zuvor beschrieben, über den Anschluss 80 eingeführte Reinigungsflüssigkeit sich in den Raum zwischen der Außenseite der Hohlwelle 10 und der Innenwand 78 verteilen, über die mäanderförmigen Schlitze 22 in der Wandung der Hohlwelle 10 radial nach innen durchtreten und diese dadurch reinigen.

Der Durchmesser 86 der Öffnung 84 in der Kappe 82 ist nunmehr so bemessen, dass er geringfügig, um Bruchteile von Millimetern, größer ist als der Außendurchmesser 17 der Hohlwelle 10, jedoch geringer ist als der Innendurchmesser 79 des Hohlraumes 74.

Dadurch entsteht um die Außenseite der Hohlwelle 10 herum im Bereich der Kappe 82 ein verengter Ringspalt 88.

Dies eröffnet die Möglichkeit, dass Reinigungsflüssigkeit über diesen Ringspalt 88 über die Außenseite der Hohlwelle 10 strömend zur Außenseite austreten kann.

Damit dies genau definiert stattfindet, ist in der Kappe 82 ein Anschlag 90 vorgesehen, an den ein entsprechender Anlageflansch 92 der Hohlwelle 10 zum Liegen kommen kann. Entweder im Anschlag 90 oder in dem Anlageflansch 92 sind axiale Einkerbungen vorgesehen, wodurch definierte Austrittsöffnungen für die Reinigungsflüssigkeit geschaffen sind, um diese vom Ringspalt 88 zur Außenseite hin austreten zu lassen. Beim Reinigungsvorgang kann nun eine gewisse Menge an Reinigungsflüssigkeit in axialer Richtung längs des Ringspaltes 88 zur Außenseite hin strömen und dabei die Außenseite reinigen. Diese zu reinigende Flüssigkeit kann dann zwischen dem Anschlag 90 und dem Anlageflansch 92 durch die besagten Einkerbungen bzw. Austrittsöffnungen hindurch definiert austreten. Somit kann die gesamte Außenseite auch im Bereich der Strömungswiderstandsmittel, also des Ringspaltes 88, gereinigt werden.

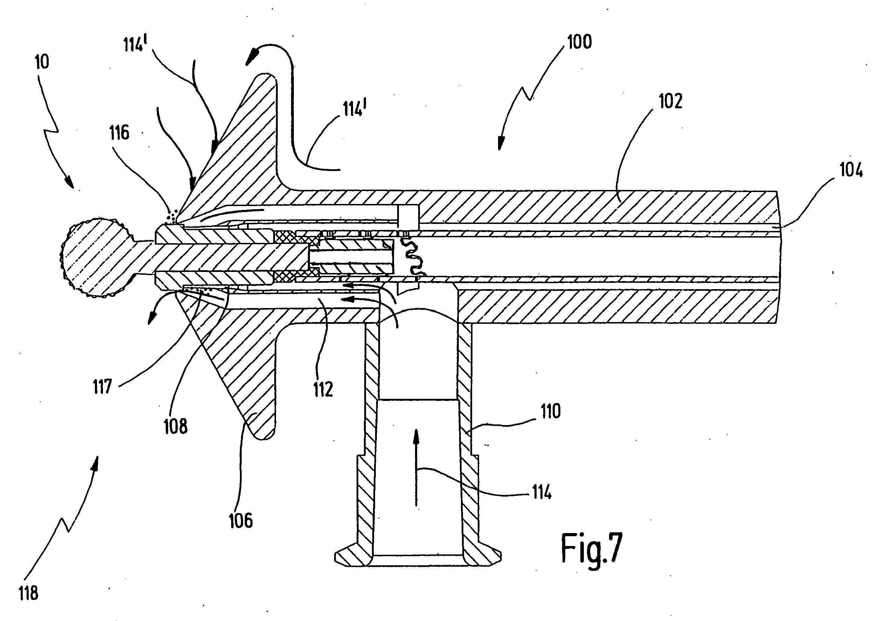

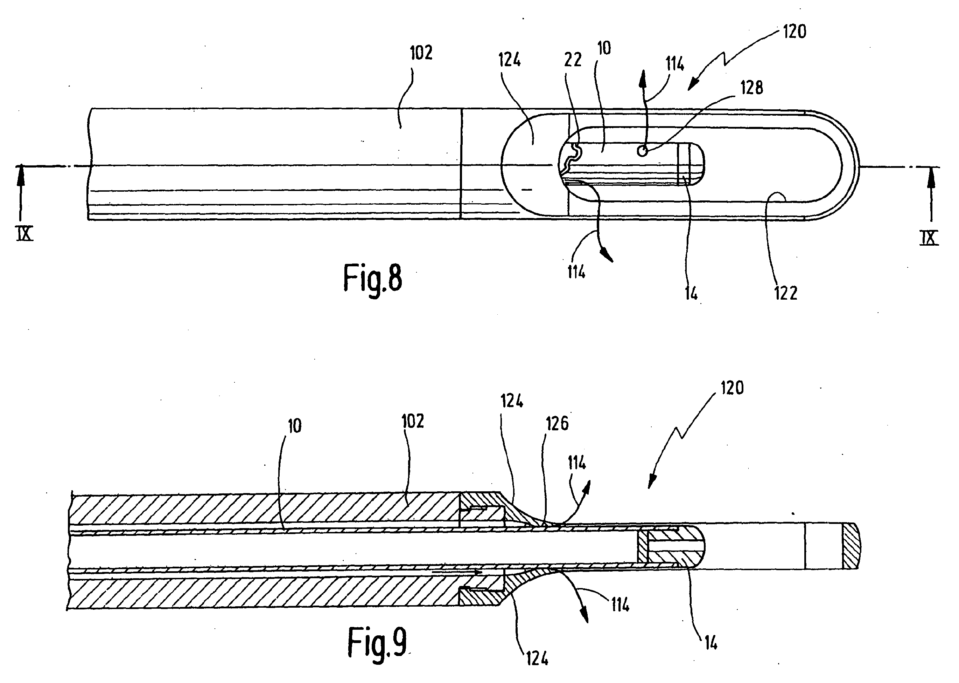

In Fig. 6 ist eine erste, hier linke Seite 94 der Vorrichtung 70 dargestellt. Die Ausgestaltung der dieser Seite gegenüberliegenden Endseite wird nachfolgend im Zusammenhang mit Fig. 8 und 9 beschrieben, da diese Ausgestaltung gleich ist wie die der nachfolgend anhand von Fig. 7 beschriebenen weiteren Ausgestaltung.

Bei der in Fig. 7 beschriebenen weiteren Ausgestaltung ist die Vorrichtung in ihrer Gesamtheit mit der Bezugsziffer 100 versehen.

Die Vorrichtung 100 weist, wie zuvor im Zusammenhang mit Fig. 6 beschrieben, einen Körper 102 auf, in dessen Innerem ein Hohlraum 104 vorgesehen ist. Der Körper 102 ist endseitig einstückig mit einer Kappe 106 versehen, die gleichermaßen wie die zuvor im Zusammenhang mit Fig. 6 beschriebe Kappe 82 ausgebildet ist. Somit ist auch hier zwischen der Außenseite einer in den Körper 102 eingeschobenen Hohlwelle 10 im Bereich der Kappe 106 ein Ringspalt 108 vorhanden, über den Reinigungsflüssigkeit 114, die über einen Stutzen 110 zugeführt wird, axial austreten kann. Beim Einführen einer an ihrer Außenseite mit Verunreinigungen versehenen Hohlwelle 10 in die Vorrichtung 100 kann nicht ausgeschlossen werden, dass gewisse Anteile der Verunreinigungen 117 in das Innere des Ringspaltes 108 eingebracht werden. Ein erheblicher Teil der an der Außenseite der Hohlwelle 10 anhaftenden Verunreinigungen 116 sammelt sich im Bereich der gekennzeichneten Stelle an, diese werden quasi über die Länge der Hohlwelle nach links abgestreift und liegen dort als umfänglicher Ring an Verunreinigungen 116 an.

Um diese Verunreinigungen 117 und 116 entfernen zu können, sind nun die nachfolgenden Maßnahmen vorgesehen.

Zum einen ist im Inneren des Körpers 102 vom Anschluss 110 abzweigend eine Bypassleitung 112 vorgesehen, die im Bereich des Ringspaltes 108 mündet. Durch den Bypass 112 kann nun ganz gezielt Reinigungsflüssigkeit 114 dieser kritischen Stelle zugeführt werden, und diese im Bereich des Ringspaltes angesammelten Verunreinigungen 117 können definiert zur Außenseite abgespült werden, wie das durch einen Strömungspfeil angedeutet ist. Diese Verunreinigungen 117 können ursprünglich beim Einschieben eingebrachte Verunreinigungen sein, oder auch Verunreinigungen, die von Reinigungsflüssigkeit in axialer Richtung längs der Außenseite axial nach außen in Richtung des Ringspaltes 108 während des Reinigungsvorganges transportiert wurden.

Durch die pilzkopf- oder kegelstumpfartige Ausgestaltung der Kappe 106 bzw. der Kappe 82 sind flüssigkeitsleitende Merkmale an der Außenseite der Vorrichtung 70 bzw. 100 geschaffen, über die es möglich ist, Reinigungsflüssigkeit 114' gezielt dem kritischen Bereich zuzuführen, in dem sich die Verunreinigungen 116 angesammelt haben.

Wie erwähnt, wird die Vorrichtung 110 üblicherweise nicht nur über den Anschluss 80 bzw. 110 im Inneren mit Reinigungsflüssigkeit 114 beaufschlagt, sondern auch an deren Außenseite mit beispielsweise aus einer vollautomatischen Waschmaschine zugeführter Waschflüssigkeit 114'. Diese von außen zugeführte Waschflüssigkeit 114' wird nun gezielt der kritischen Stelle zugeführt und spült auch diese Verunreinigungen 116 ab. Durch die relativ großen Flächen wird auch die Flüssigkeitsmenge erhöht.

In den Fig. 8 und 9 ist die zweite Seite 120 der in den Fig. 6 bzw. 7 dargestellten ersten Seiten 94 und 118 der Vorrichtungen 70 und 100 dargestellt.

Bei beiden Vorrichtungen 70 und 100 ist diese zweite Seite gleich ausgestaltet.

Aus Fig. 8 ist zu entnehmen, dass der Körper jeweils an der zweiten Seite 120 mit einer nadelöhrartigen Öffnung 122 versehen ist, in die der Körper 102 über eine beidseitige Verjüngung 124 übergeht.

Im Bereich der Verjüngung 124 existiert um die Außenseite der eingeschobenen Hohlwelle 120 ein Ringspalt 126, über den Reinigungsflüssigkeit 114 austreten kann, wie das durch die Strömungspfeile angedeutet ist.

Die Konstruktion des Ringspaltes 126 ist gleich den Ringspälten 88 bzw. 108, so dass auch hier in diesem Bereich der Strömungswiderstandsmittel eine kontinuierliche Spülung der Außenseite der Hohlwelle 10 stattfinden kann.

Wenn an dem proximalen Ende 14 der Hohlwelle 10, die in dem Bereich der Öffnung 122 endet, noch Teile des mäanderförmigen Schlitzes 22 liegen, kann über diesen Reinigungsflüssigkeit aus der Innenseite der Hohlwelle 10 austreten. Dies kann aber auch über eine extra dazu vorgesehene Austrittsöffnung 128 am proximalen Ende 14 der Hohlwelle 10 erfolgen, so dass eine ganz gezielte Austrittsöffnung vorhanden ist, über die Reinigungsflüssigkeit 114 austreten kann.

Bei den in den Fig. 6 bis 9 beschriebenen Ausgestaltungen sind jeweils geradlinig erstreckende Vorrichtungen bzw. deren Körper 72 und 102 beschrieben.

Auch hier ist es prinzipiell möglich, den Körper gekrümmt auszubilden und gegebenenfalls die Hohlwelle im Körper zu drehen. Dies kann dann erwünscht sein, wenn zu befürchten ist, dass sich in den mäanderförmigen Schlitzen 22 keilförmige Partikel festgesetzt haben, die durch die von radial außen nach innen in die Hohlwelle eintretende Reinigungsflüssigkeit nicht mitgerissen werden können. Wie zuvor erwähnt, kann durch die Krümmung und durch Drehen jeweils ein kurzzeitiges Erweitern der Schlitze im äußeren Krümmungsbereich erfolgen, so dass dann diese Festteile nach innen abgespült und beispielsweise zentral über eine Öffnung 128, wie in Fig. 8 dargestellt, abgeführt werden. Da das proximale Ende 14 in der Öffnung 122 freiliegend ist, können alle Verunreinigungen laufend abgespült werden, beispielsweise wenn die Vorrichtung, wie zuvor beschrieben, in einer Wasch-/Spülmaschine aufgenommen ist, über die nicht nur die Innenseite der Vorrichtung mit Reinigungsflüssigkeit beaufschlagt wird, sondern auch deren Außenseite. Je nach Ausgestaltung können dann selbstverständlich mehrere derartige Vorrichtungen gleichzeitig betrieben und mit Reinigungsflüssigkeit beaufschlagt werden.

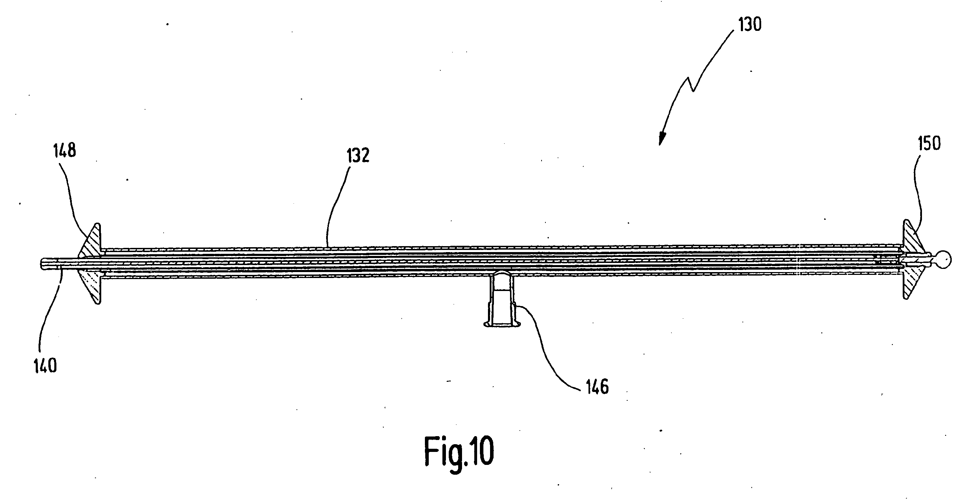

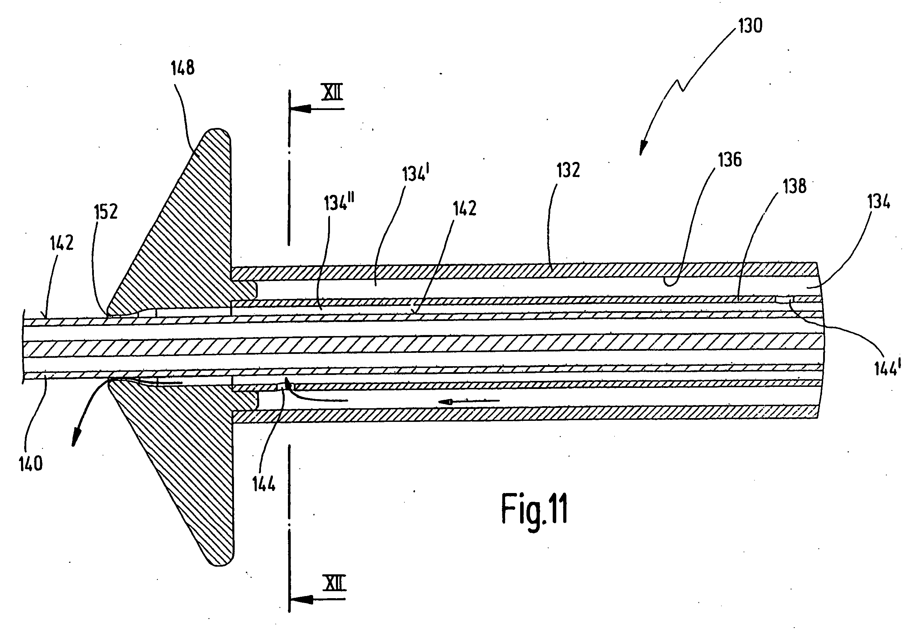

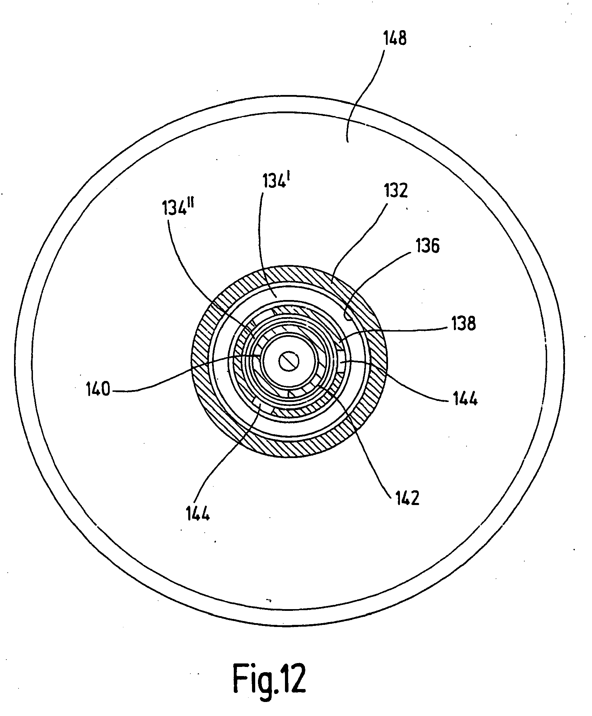

Bei einem in den Figuren 10 bis 12 dargestellten weiteren Ausführungsbeispiel einer erfindungsgemäßen Vorrichtung 130 weist diese ebenfalls einen langerstreckten, geradlinigen, hohlzylindrischen Körper 132 auf, an dessen beiden Enden jeweils Kappen 148 und 150 angeordnet sind. Im Hohlraum 134 ist im Gegensatz zu den zuvor beschriebenen Ausführungen noch zusätzlich eine rohrförmige bzw. hohlzylindrische Zwischenwand 138 vorgesehen. Die Außenseite der Zwischenwand 138 befindet sich im Abstand zu der Innenwand 136 des Hohlraumes 134.

Der lichte Innendurchmesser der Zwischenwand 138 ist größer als der Außendurchmesser der Hohlwelle 140. Dadurch befindet sich die Außenseite 142 der Hohlwelle 140 in radialem Abstand zur Zwischenwand 138.

Die Zwischenwand 138 teilt somit den Hohlraum 134 in einen äußeren Hohlraum 134' und in einen inneren Hohlraum 134" auf. In der Zwischenwand 138 sind zahlreiche Durchtrittsöffnungen 144 ausgespart.

Wird dem Anschluss 146 wie zuvor beschrieben das Reinigungsmittel, z.B. eine Reinigungsflüssigkeit, zugeführt, so strömt dies zunächst in den äußeren Bereich 134' des Hohlraumes 134 und verteilt sich dort gleichmäßig. Über die Öffnungen 144 dringt die Reinigungsflüssigkeit in den inneren Bereich 134" des Hohlraumes 134 ein. Von dort aus kann dann die Reinigungsflüssigkeit über den zuvor beschriebenen mäanderförmigen Schlitz in das Innere der Hohlwelle 140 eintreten.

Wie zuvor im Zusammenhang mit den Ausgestaltungen von Fig. 6 und 7 beschrieben, besteht im Endbereich der Kappen 148 und 150 zur Außenseite 142 der Hohlwelle 140 ein schmaler Spalt 152, über den Reinigungsflüssigkeit austreten kann.

Insbesondere im endseitigen Bereich der Zwischenwand 138 sind Öffnungen 144 angeordnet, um genau diesen Bereich mit ausreichend unter Druck stehendem Reinigungsmittel zu versorgen.

## Patentansprüche

1. Vorrichtung zum Reinigen einer flexiblen Hohlwelle (10, 140) eines medizinischen Instrumentes, wobei die Vorrichtung einen Hohlraum (34, 74, 104, 134) aufweisenden Körper (32, 72, 102, 132) aufweist, in dem zumindest ein Abschnitt einer zu reinigenden flexiblen Hohlwelle (10, 140) aufgenommen ist, wobei die Hohlwelle (10, 140) ein proximales Ende (14), ein distales Ende (12) und eine Wandung (18) aufweist, die mit Öffnungen (20) versehen ist, wobei der Hohlraum (34, 74, 104, 134) einen Anschluss (40, 80, 110, 146) aufweist über dem ein Reinigungsmittel (45, 114) zuführbar ist, wobei eine Innenwand (38, 78, 136) des Hohlraumes (34, 74, 104, 134) sich in radialem Abstand zur Außenseite (19, 142) der Hohlwelle (10, 140) befindet, und wobei axial voneinander beabstandete Strömungswiderstandsmittel vorhanden sind, die einen Raum (58) zwischen der Außenseite (19) der Hohlwelle (10, 140) und der Innenwand (38, 78) des Hohlraumes (34, 74, 104, 134) in axialer Richtung begrenzen, **dadurch gekennzeichnet, dass** die Hohlwelle (10, 140) sowohl mit ihren proximalen als auch distalen Ende (12, 14) aus dem Raum (58) in axialer Richtung hinausragt, und dass die axial voneinander beabstandeten Strömungswiderstandsmittel so ausgestaltet sind, dass der Strömungswiderstand der Öffnungen (20) der flexiblen Hohlwelle (10, 140) bei entsprechender Druckbeaufschlagung des Reinigungsmittels, durch das Reinigungsmittel (45, 114) überwindbar ist, so dass das zugeführte Reinigungsmittel (45, 114) über die Öffnungen (20) in der Wandung (18) der Hohlwelle (10, 140) in deren Innenraum eindringen kann, wodurch zumindest ein Teil des Reinigungsmittels (45, 114) den Innenraum der Hohlwelle (10, 140) reinigt und durch die Hohlwelle (10, 140) abgeführt werden kann.

2. Vorrichtung nach Anspruch 1, **dadurch gekennzeichnet, dass** der Körper (32, 72, 102, 132) als Hohlzylinder (36) ausgebildet ist.

3. Vorrichtung nach Anspruch 1 oder 2, **dadurch gekennzeichnet, dass** der Körper (32, 72, 102, 132) geradlinig verläuft.

4. Vorrichtung nach Anspruch 1 oder 2, **dadurch gekennzeichnet, dass** der Körper gekrümmt verläuft.

5. Vorrichtung nach einem der Ansprüche 1 bis 4, **dadurch gekennzeichnet, dass** der axiale Abstand (54) der Strömungswiderstandsmittel in etwa der Länge des Abschnittes der Hohlwelle (10) entspricht, der mit den Öffnungen (20) versehen ist.

6. Vorrichtung nach einem der Ansprüche 1 bis 5, **dadurch gekennzeichnet, dass** die Strömungswiderstandsmittel als Dichtungsmittel (48, 49) ausgebildet sind.

7. Vorrichtung nach einem der Ansprüche 1 bis 6, **dadurch gekennzeichnet, dass** der Anschluss (40) etwa mittig im Körper (32) angeordnet ist.

8. Vorrichtung nach einem der Ansprüche 1 bis 7, **dadurch gekennzeichnet, dass** die Hohlwelle (10) relativ zum Körper (32) verschiebbar ist.

9. Vorrichtung nach einem der Ansprüche 6 bis 8, **dadurch gekennzeichnet, dass** der axiale Abstand (54) der Dichtungsmittel (48, 49) untereinander veränderbar ist.

10. Vorrichtung nach einem der Ansprüche 6 bis 9, **dadurch gekennzeichnet, dass** die Dichtungsmittel (48, 49) als O-Ringe (50, 51) ausgebildet sind.

11. Vorrichtung nach einem der Ansprüche 6 bis 10, **dadurch gekennzeichnet, dass** die Dichtungsmittel (48, 49) in axial verschiebbaren Lagerbuchsen aufgenommen sind.

12. Vorrichtung nach einem der Ansprüche 1 bis 5, **dadurch gekennzeichnet, dass** die Strömungswiderstandsmittel **dadurch** gebildet sind, dass der radiale Abstand der Innenwand (78) des Hohlraumes (74) zur Außenseite (19) der Hohlwelle (10) im Bereich der Strömungswiderstandsmittel geringer ist als im Bereich zwischen diesen axial voneinander beabstandeten Stellen.

13. Vorrichtung nach Anspruch 12, **dadurch gekennzeichnet, dass** im Bereich eines Strömungswiderstandsmittels ein Ringspalt (88, 108) zwischen der Innenwand (78) des Hohlraumes (74) und der Außenseite (19) der Hohlwelle (10) vorhanden ist, durch den Reinigungsmittel (114) axial durchtreten kann.

14. Vorrichtung nach Anspruch 12 oder 13, **dadurch gekennzeichnet, dass** Reinigungsmittel (114) zusätzlich dem verengten Raum bzw. dem Ringspalt (108) unmittelbar zuführbar ist.

15. Vorrichtung nach Anspruch 14, **dadurch gekennzeichnet, dass** vom Anschluss (110) zumindest ein Bypass (112) abzweigt, der im Bereich des verengten Raumes bzw. des Ringspaltes (108) mündet.

16. Vorrichtung nach einem der Ansprüche 12 bis 15, **dadurch gekennzeichnet, dass** eine zu reinigende Hohlwelle (10) nur von einer ersten Seite (94, 118) her in den Hohlraum (74, 104) einführbar ist.

17. Vorrichtung nach Anspruch 16, **dadurch gekennzeichnet, dass** an der ersten Seite (94) ein Anschlag (90) vorhanden ist, an den eine entsprechende Anlage (92) einer Hohlwelle (10) anlegbar ist.

18. Vorrichtung nach Anspruch 16 oder 17, **dadurch gekennzeichnet, dass** an der ersten Seite (94, 118) ein distales Ende (12) der Hohlwelle (10) zum Liegen kommt.

19. Vorrichtung nach einem der Ansprüche 16 bis 18, **dadurch gekennzeichnet, dass** der Körper (102) an einer der ersten Seite (94, 118) gegenüberliegenden zweiten Seite (120) eine Öffnung (122) aufweist, in deren Bereich das proximale Ende (14) der Hohlwelle (10) zum Liegen kommt.

20. Vorrichtung nach einem der Ansprüche 12 bis 19, **dadurch gekennzeichnet, dass** der Körper (72, 102) zumindest im Bereich einer ersten Seite (94, 128) an dessen Außenseite flüssigkeitsleitende Merkmale aufweist, die auf die Außenseite auftreffende Reinigungsflüssigkeit (114') gezielt auf die erste Seite (94, 118) zuführen.

21. Vorrichtung nach einem der Ansprüche 1 bis 20, **dadurch gekennzeichnet, dass** im Hohlraum (134) im Abstand zu dessen Innenwand (136) eine Zwischenwand (138) angeordnet ist, die im Abstand zur Außenseite (142) der Hohlwelle (140) liegt, und wobei in der Zwischenwand (138) Durchtrittsöffnungen (144) vorhanden sind.

## Claims

1. Device for cleaning a flexible hollow shaft (10, 140) of a medical instrument, the device having a body (32, 72, 102, 132) comprising a hollow space (34, 74, 104, 134) in which at least a section of a hollow shaft (10, 140) to be cleaned is housed, wherein the hollow shaft (10, 140) has a proximal end (14), a distal end (12) and a wall (18) provided with openings (20), wherein the hollow space (34, 74, 104, 134) has an attachment piece (40, 80, 110, 146) via which a cleaning agent (45, 144) can be delivered, wherein an inside wall (38, 78, 136) of the hollow space (34, 74, 104, 134) is at a radial distance from the outer face (19, 142) of the hollow shaft (10, 140), and wherein flow resistance means, spaced axially apart from one another, are provided which delimit, in the axial direction, a space (58) between the outer face (19) of the hollow shaft (10, 140) and the inside wall (38, 78) of the hollow space (34, 74, 104, 134), **characterized in that** the hollow shaft (10, 140) extends with both, its proximal and its distal ends (12, 14) beyond the space (58) in axial direction, and **in that** the axially spaced apart flow resistance means are designed **in that** the flow resistance of the openings (20) of the flexible hollow shaft (10, 140) can be overcome by the cleaning agent (45, 114) with a respective pressure charge of the cleaning agent, so that the delivered cleaning agent (45, 114) can penetrate via the openings (20) in the wall (18) of the hollow shaft (10, 140) into its inner space, wherein at least a part of the cleaning agent (45, 114) cleans the inner space of the hollow shaft (10, 140) and can be discharged via the hollow shaft (10, 140).

2. Device of claim 1, **characterized in that** the body (32, 72, 102, 132) is designed as a hollow cylinder (36).

3. Device of claims 1 or 2, **characterized in that** the body (32, 72, 102, 132) extends in a straight line.

4. Device of claims 1 or 2, **characterized in that** the body extends in a curved shape.

5. Device of anyone of claims 1 through 4, **characterized in that** the axial distance (54) between the flow resistance means corresponds approximately to the length of that section of the hollow shaft (10) provided with the openings (20).

6. Device of anyone of claims 1 through 5, **characterized in that** the flow resistance means are designed as sealing means (48, 49).

7. Device of anyone of claims 1 through 6, **characterized in that** the attachment piece (40) is arranged approximately centrally in the body (32).

8. Device of anyone of claims 1 through 7, **characterized in that** the hollow shaft (10) is displaceable relative to the body (32).

9. Device of anyone of claims 6 through 8, **characterized in that** the axial distance (54) between the sealing means (48, 49) is variable.

10. Device of anyone of claims 6 through 9, **characterized in that** the sealing means (48, 49) are designed as 0-rings (50, 51).

11. Device of anyone of claims 6 through 10, **characterized in that** the sealing means (48, 49) are received in axially displaceable bearing bushings.

12. Device of anyone of claims 1 through 5, **characterized in that** the flow resistance means are formed by means of the fact that the distance between the inside wall (78) of the hollow space (74) and the outer face (19) of the hollow shaft (10) is smaller in the area of the flow resistance means than it is in the area between these axially spaced-apart locations.

13. Device of claim 12, **characterized in that** in the area of a flow resistance means, an annular gap (88, 108) is present between the inside wall (78) of the hollow space (74) and the outer face (19) of the hollow shaft (10), and wherein cleaning agent (114) can pass axially through this annular gap (88, 108).

14. Device of claims 12 or 13, **characterized in that** the cleaning agent (114) can additionally be delivered directly to the narrowed space or annular gap (108).

15. Device of claim 14, **characterized in that** at least one bypass (112), branching off from the attachment piece (110), opens out in the area of the narrowed space or annular gap (108).

16. Device of anyone of claims 12 through 15, **characterized in that** a hollow shaft (10) to be cleaned can be introduced into the hollow space (74, 104) only from a first end (94, 118).

17. Device of claim 16, **characterized in that** a limit stop (90) is provided at the first end (94), onto which a respective abutment (92) of a hollow shaft (10) can be placed.

18. Device of claims 16 or 17, **characterized in that** a distal end (12) of the hollow shaft (10) comes to lie on the first end (94, 118).

19. Device of anyone of claims 16 through 18, **characterized in that** the body (102) at a second end (120) lying remote from the first end (94, 118) has an opening (122), in the area of which the proximal end (14) of the hollow shaft (10) comes to lie.

20. Device of anyone of claims 12 through 19, **characterized in that** the body (72, 102), at least in an area of a first end (94, 118) is provided on its outer face with liquid-conveying features, by which cleaning liquid (114') impinging on the outer face is delivered in a target manner to the first end (94, 118).

21. Device of anyone of claims 1 through 20, **characterized in that** the hollow space (134) contains, at a distance from its inside wall (136) a partition wall (138) which lies at a distance from the outer face (142) of the hollow shaft (140), and **in that** through-openings (144) are provided in the partition wall (138).

## Revendications

1. Dispositif affecté au nettoyage d'un arbre creux flexible (10, 140) d'un instrument médical, ledit dispositif comportant un corps (32, 72, 102, 132) qui est muni d'une cavité (34, 74, 104, 134) et dans lequel est logé au moins un tronçon d'un arbre creux flexible (10, 140) devant être nettoyé, lequel arbre creux (10, 140) offre une extrémité proximale (14), une extrémité distale (12) et une paroi (18) percée d'orifices (20), la cavité (34, 74, 104, 134) présentant un raccord (40, 80, 110, 146) par l'intermédiaire duquel un agent nettoyant (45, 114) peut être délivré, sachant qu'une paroi intérieure (38, 78, 136) de ladite cavité (34, 74, 104, 134) se trouve à distance radiale de la face extérieure (19, 142) de l'arbre creux (10, 140), et sachant que des moyens de résistance à l'écoulement axialement distants les uns des autres délimitent, dans le sens axial, un espace (58) entre ladite face extérieure (19) de l'arbre creux (10, 140) et ladite paroi intérieure (38, 78) de la cavité (34, 74, 104, 134), **caractérisé par le fait que** l'arbre creux (10, 140) fait saillie au-delà de l'espace (58) dans le sens axial, par ses extrémités (14, 12) tant proximale, que distale ; et **par le fait que** les moyens de résistance à l'écoulement, axialement distants les uns des autres, sont agencés de façon telle que la résistance à l'écoulement, opposée par les orifices (20) de l'arbre creux flexible (10, 140), puisse être surmontée par l'agent nettoyant (45, 114) lors d'une pressurisation correspondante dudit agent nettoyant, de sorte que l'agent nettoyant délivré (45, 114) peut pénétrer dans l'espace interne dudit arbre creux (10, 140), en empruntant les orifices (20) pratiqués dans la paroi (18) de ce dernier, si bien qu'au moins une part dudit agent nettoyant (45, 114) nettoie ledit espace interne de l'arbre creux (10, 140), et peut être évacuée à travers ledit arbre creux (10, 140).

2. Dispositif selon la revendication 1, **caractérisé par le fait que** le corps (32, 72, 102, 132) est réalisé sous la forme d'un cylindre creux (36).

3. Dispositif selon la revendication 1 ou 2, **caractérisé par le fait que** le corps (32, 72, 102, 132) présente un tracé rectiligne.

4. Dispositif selon la revendication 1 ou 2, **caractérisé par le fait que** le corps présente un tracé curviligne.

5. Dispositif selon l'une des revendications 1 à 4, **caractérisé par le fait que** la distance axiale (54), séparant les moyens de résistance à l'écoulement, correspond approximativement à la longueur du tronçon de l'arbre creux (10) qui est muni des orifices (20).

6. Dispositif selon l'une des revendications 1 à 5, **caractérisé par le fait que** les moyens de résistance à l'écoulement sont réalisés sous la forme de moyens d'étanchement (48, 49).

7. Dispositif selon l'une des revendications 1 à 6, **caractérisé par le fait que** le raccord (40) occupe une position approximativement centrale dans le corps (32).

8. Dispositif selon l'une des revendications 1 à 7, **caractérisé par le fait que** l'arbre creux (10) peut coulisser vis-à-vis du corps (32).

9. Dispositif selon l'une des revendications 6 à 8, **caractérisé par le fait que** la distance axiale (54), séparant mutuellement les moyens d'étanchement (48, 49), peut être modifiée.

10. Dispositif selon l'une des revendications 6 à 9, **caractérisé par le fait que** les moyens d'étanchement (48, 49) sont réalisés sous la forme de bagues toriques (50, 51).

11. Dispositif selon l'une des revendications 6 à 10, **caractérisé par le fait que** les moyens d'étanchement (48, 49) sont reçus par des douilles de montage aptes à coulisser dans le sens axial.

12. Dispositif selon l'une des revendications 1 à 5, **caractérisé par le fait que** la formation des moyens de résistance à l'écoulement résulte du fait que la distance radiale comprise entre la paroi intérieure (78) de la cavité (74) et la face extérieure (19) de l'arbre creux (10) est moindre, dans la région desdits moyens de résistance à l'écoulement, que dans la région située entre ces zones axialement distantes les unes des autres.

13. Dispositif selon la revendication 12, **caractérisé par le fait qu'**un interstice annulaire (88, 108), par lequel de l'agent nettoyant (114) peut circuler dans le sens axial, est réservé entre la paroi intérieure (78) de la cavité (74) et la face extérieure (19) de l'arbre creux (10), dans la région d'un moyen de résistance à l'écoulement.

14. Dispositif selon la revendication 12 ou 13, **caractérisé par le fait que** de l'agent nettoyant (114) peut être respectivement délivré, additionnellement et en mode direct, à l'espace rétréci ou à l'interstice annulaire (108).

15. Dispositif selon la revendication 14, **caractérisé par le fait qu'**au moins une dérivation (112), bifurquant du raccord (110), débouche respectivement dans la région de l'espace rétréci ou de l'interstice annulaire (108).

16. Dispositif selon l'une des revendications 12 à 15, **caractérisé par le fait qu'**un arbre creux (10) devant être nettoyé peut être introduit, dans la cavité (74, 104), uniquement à partir d'un premier côté (94, 118).

17. Dispositif selon la revendication 16, **caractérisé par** la présence, sur le premier côté (94), d'une butée (90) contre laquelle peut être plaquée une zone de contact correspondante (92) d'un arbre creux (10).

18. Dispositif selon la revendication 16 ou 17, **caractérisé par le fait qu'**une extrémité distale (12) de l'arbre creux (10) vient en applique contre le premier côté (94, 118).

19. Dispositif selon l'une des revendications 16 à 18, **caractérisé par le fait que** le corps (102) présente, sur un second côté (120) opposé au premier côté (94, 118), une ouverture (122) dans la région de laquelle l'extrémité proximale (14) de l'arbre creux (10) vient en applique.

20. Dispositif selon l'une des revendications 12 à 19, **caractérisé par le fait que** le corps (72, 102) est doté à sa face extérieure, au moins dans la région d'un premier côté (94, 118), de caractéristiques de canalisation de fluides qui dirigent adéquatement, vers ledit premier côté (94, 118), du fluide de nettoyage (114') venant rencontrer ladite face extérieure.

21. Dispositif selon l'une des revendications 1 à 20, **caractérisé par le fait qu'**une cloison intermédiaire (138), située à distance de la face extérieure (142) de l'arbre creux (140), est logée dans la cavité (134) à distance de la paroi intérieure (136) de cette dernière, des orifices de passage (144) étant pratiqués dans ladite cloison intermédiaire (138).
